# FASCICULE DE BREVET EUROPEEN

(11) **EP 2 328 599 B1**
(45) Date de publication et mention de la délivrance du brevet: **20.06.2012**
(21) Numéro de dépôt: 09786230.4
(22) Date de dépôt: 08.07.2009
(51) Int. Cl.: A61K 36/47, A61P 9/12

(54) **COMPOSITION ANTI-HYPERTENSIVE CONTENANT UN EXTRAIT D'HYMENOCARDIA ACIDA**
ANTIHYPERTENSIVE ZUSAMMENSETZUNG MIT EINEM EXTRAKT AUS HYMENOCARDIA ACIDA
ANTIHYPERTENSIVE COMPOSITION CONTAINING AN EXTRACT OF HYMENOCARDIA ACIDA

(30) Priorité: 30.07.2008 FR 0804350
(43) Date de publication de la demande: 08.06.2011
(73) Titulaire: Balde, Aliou Mamadou, Conakry I (GU)
(72) Inventeur: Balde, Aliou Mamadou, Conakry I (GU)
(74) Mandataire: Metz, Paul
(86) Numéro de dépôt international: PCT/IB2009/006783
(87) Numéro de publication internationale: WO 2010/013144

(56) Documents cités:
- UKWE C V: "Antiulcer activity of aqueous stembark extract of Hymenocardia acida Tul (Euphorbiaceae)" INTERNATIONAL JOURNAL OF PHARMACOGNOSY, SWETS & ZEITLINGER, LISSE, NL, vol. 35, no. 5, 1 décembre 1997 (1997-12-01), pages 354-357, XP009112653 ISSN: 0925-1618
- HOET SARA ET AL: "In vitro antitrypanosomal activity of ethnopharmacologically selected Beninese plants." JOURNAL OF ETHNOPHARMACOLOGY, vol. 91, no. 1, mars 2004 (2004-03), pages 37-42, XP002517371 ISSN: 0378-8741
- VONTHRON-SENECHEAU C ET AL: "In vitro antiplasmodial activity and cytotoxicity of ethnobotanically selected Ivorian plants" 20041001, vol. 26, no. 5, 1 octobre 2004 (2004-10-01), XP018001862
- JOHN O. IGOLI ET AL.: "Friedelanone and other triterpenoids from Hymenocardia acida" INTERNATIONAL JOURNAL OF PHYSICAL SCIENCES, vol. 3, no. 6, juin 2008 (2008-06), pages 156-158, XP002517372

## Description

La présente invention concerne l'utilisation d'un extrait végétal d'Hymenocardia acida dans la formulation d'une composition pharmaceutique utile pour la prévention et le traitement de l'hypertension.

Elle concerne aussi une composition pharmaceutique anti-hypertensive à base d'extraits végétaux, qui contient parmi ses principes actifs un extrait végétal d'Hymenocardia acida.

Avantageusement, la composition anti-hypertensive de l'invention peut contenir également une quantité adaptée de sélénium.

L'hypertension touche plus de 15% de la population des pays occidentaux et de plus en plus de personnes dans les pays émergents. Au-dessus de l'âge de 65 ans, sa fréquence passe à environ 35% de la population. L'hypertension, qui se traduit par une pression sanguine trop élevée, est une cause reconnue des maladies cardiovasculaires. Elle peut provoquer des accidents vasculaires cérébraux, une hypertrophie du cεur, une insuffisance cardiaque congestive, de l'artériosclérose, des dommages aux reins ou aux yeux et peut conduire à une mort prématurée.

De nombreuses études ont montré que lorsque l'on réussit à faire baisser, même faiblement, la pression sanguine des patients souffrant d'hypertension, les risques liés à ces maladies diminuent. Il est donc très important de réguler l'hypertension par un traitement approprié.

Cependant et malgré la gravité des conséquences potentielles, de nombreux patients atteints d'hypertension ne suivent aucun traitement et ne sont parfois même pas conscients dé leur état.

La raison principale est qu'il est difficile pour beaucoup de patients de se conformer aux traitements actuels de l'hypertension. Ces traitements, généralement à base de bêtabloquants, sont uniquement des traitements symptomatiques. Ce qui signifie qu'une fois qu'ils ont commencé à prendre ces médicaments, les patients doivent continuer tout au long de leur vie. Il est même dangereux d'arrêter le traitement une fois commencé, du fait du brusque changement de pression sanguine occasionné qui peut causer de graves dommages.

Or, ces médicaments classiques ont de nombreux effets secondaires gênants, parmi lesquels on peut citer la somnolence, l'augmentation des taux de triglycérides et de cholestérol, l'impuissance, la dépressionβ

Pour toutes ces raisons, les patients et surtout ceux atteints d'une hypertension légère ou modérée, hésitent avant de débuter un traitement.

Un traitement efficace contre l'hypertension, mais ne présentant pas tous ces effets secondaires est donc fortement souhaité.

Un autre problème, qui vient s'ajouter à ceux précédemment cités, est le coût élevé des traitements classiques contre l'hypertension qui rend difficile l'accès aux soins dans les pays en voie de développement. Un traitement par ces spécialités pharmaceutiques a un prix de revient de l'ordre de 30 à 40□ par mois qui est difficilement supportable à long terme par la population de ces pays. L'accès au traitement contre l'hypertension est donc limité à une très faible part des personnes concernées.

Dans ces pays, notamment en Afrique, il serait particulièrement intéressant de disposer d'un traitement efficace et beaucoup moins cher, obtenu à partir de plantes locales.

C'est le but recherché par l'invention dont l'objectif est de fournir un médicament efficace, avec beaucoup moins d'effets secondaires et dont le coût resterait préférentiellement inférieur à 5□ par mois.

Pour résoudre ce problème technique, l'invention enseigne une composition pharmaceutique anti-hypertensive, utile pour la prévention et le traitement de l'hypertension, réalisée à partir d'extraits végétaux.

Selon l'invention, elle contient parmi ses principes actifs un extrait végétal d'Hymenocardia acida.

De préférence, elle peut également contenir du sélénium.

Avantageusement, la composition selon l'invention ne présente pas ou très peu d'effets secondaires et est beaucoup moins chère que les médicaments classiques contre l'hypertension. Elle est ainsi bien acceptée par les patients pour un traitement régulier de longue durée.

Elle est particulièrement efficace dans les cas d'hypertension faible et modérée correspondant aux stades 1 et 2 de la classification, mais peut également être utilisée dans les cas d'hypertension forte de stade 3.

Elle a un rôle normo-régulateur qui permet au patient d'éviter de passer à un stade supérieur plus grave d'hypertension ou du moins qui ralentit cette évolution en prolongeant la durée des stades inférieurs.

Elle peut également avantageusement servir à traiter les cas de pré-hypertension, c'est-à-dire les patients dont la pression sanguine présente une valeur correspondant ou proche de la limite inférieure fixée pour définir l'hypertension faible, qui n'étaient pas traités jusqu'à présent en raison des nombreux inconvénients des traitements classiques.

D'autres caractéristiques et avantages de l'invention apparaîtront à la lecture de la description détaillée qui va suivre, description faite en référence aux dessins annexés, dans lesquels :
- la figure 1 est un graphique illustrant en comparaison les variations de la tension diastolique, en suivi hospitalier, d'un groupe traité avec Hymenocardia acida et d'un groupe traité avec un placebo ;
- la figure 2 est un graphique illustrant en comparaison les variations de la tension systolique, en suivi à domicile, d'un groupe traité avec Hymenocardia acida et d'un groupe traité avec un placebo ;
- la figure 3 est un graphique illustrant en comparaison les variations de la pression pulsée, en suivi hospitalier, d'un groupe traité avec Hymenocardia acida et d'un groupe traité avec un placebo ;
- la figure 4 est un graphique illustrant en comparaison les variations de la pulsation, en suivi à domicile, d'un groupe traité avec Hymenocardia acida et d'un groupe traité avec un placebo.

Hymenocardia acida est une plante guinéenne largement distribuée dans les savanes boisées et les bordures marginales entre la savane et la grande forêt. Elle se présente sous la forme d'un arbuste de 5 à 8 mètres à branches et fût tortueux et dont les feuilles facilement reconnaissables sont couvertes de petits points glandulaires jaune-orangés.

Des peuplements spontanés importants se rencontrent en Moyenne-Guinée, en Haute-Guinée et dans une moindre mesure en Basse-Guinée.

Pour réaliser la composition selon l'invention, on utilise un extrait végétal d'Hymenocardia acida. Cet extrait est préférentiellement obtenu à partir des feuilles d'Hymenocardia acida qui ne contiennent pas d'alcaloïdes et ne présentent donc que très peu d'effets secondaires.

Il s'agit préférentiellement d'une solution végétale ou d'un extrait végétal liquide et par exemple selon sa concentration d'une teinture mère, d'une teinture officinale ou d'un extrait liquide d'Hymenocardia acida.

Cette solution ou extrait liquide est obtenu par tout moyen approprié et notamment par extraction, préférentiellement aqueuse, alcoolique ou hydro-alcoolique, par macération, infusion, décoction, digestion, lixiviation ou analogue.

Selon un mode de réalisation préférentiel de l'invention, on prépare un extrait végétal liquide à partir de feuilles d'Hymenocardia acida au moyen d'une extraction hydro-alcoolique par une technique de percolation avec agitation. Sa teneur en alcool est préférentiellement comprise entre 30 et 70°.

A titre d'exemple, on a préparé une teinture mère d'Hymenocardia acida avec une teneur en éthanol d'environ 70% V/V, à partir de feuilles sèches d'Hymenocardia acida par percolation exhaustive à froid suivie d'une concentration sous vide à l'évaporateur rotatif.

L'extrait obtenu est un liquide brun foncé d'odeur caractéristique.

Il contient de nombreux principes actifs dont plusieurs ont été identifiés et notamment des glycosides flavoniques et des proanthocyanidines.

Une analyse qualitative de la teinture mère précédemment décrite par chromatographie sur couche mince et révélation à la vanilline sulfurique a démontré la présence d'isovitexine et d'iso-orientine.

Afin d'étudier plus complètement la composition chimique des feuilles d'Hymenocardia acida une série d'analyses a été réalisée à partir de 100g de poudre de feuilles d'Hymenocardia acida.

Une extraction au chloroforme de cette poudre de feuilles a d'abord permis de séparer un extrait lipophile (3.65 g) d'un premier marc. On a ensuite procédé à une extraction au méthanol de ce premier marc qui a conduit à un extrait polaire (6,15 g) et à un deuxième marc.

L'extrait lipophile a été soumis à une chromatographie sur colonne, répétitive et éluée avec un mélange de chloroforme et de méthanol selon un gradient de polarité. Un mélange de stérols, dont le □-sitostérol et des acides gras saturés ont été isolés des premières fractions non polaires résultants de cette chromatographie. Ils ont été identifiés par résonance magnétique nucléaire du proton et du carbone.

L'extrait polaire a également été soumis à une chromatographie sur colonne, répétitive et éluée avec de l'éthanol. On a ainsi isolé un mélange de 2C-glycosylflavones (45 mg) qui ont été identifiées comme étant de l'iso-orientine et de l'isovitexine, avec un ratio de 1 pour 4 respectivement, par spectroscopie de résonance magnétique nucléaire du proton et du carbone et par spectroscopie de masse.

Des proanthocyanidines ont également été mises en évidence dans cet extrait polaire par chromatographie sur couche mince et révélation à la vanilline sulfurique.

Par ailleurs, plusieurs alcaloïdes dont l'hyménocardine ont été détectés dans les écorces de racine d'Hymenocardia acida. Pour cette raison, on utilise préférentiellement les feuilles d'Hymenocardia acida, qui ne contiennent pas d'alcaloïdes, pour réaliser la composition anti-hypertensive de l'invention.

La composition selon l'invention peut adopter différentes formes galéniques. Elle peut ainsi par exemple se présenter sous la forme d'infusettes contenant par exemple dix à vingt grammes de feuilles d'Hymenocardia acida grossièrement broyées.

Elle peut également se présenter sous la forme de comprimés, préférentiellement orodispersibles, de capsules molles, de granules, de gélules, de poudre, d'ampoules, de sirop ou de toute autre forme galénique appropriée, préférentiellement orale, imaginable par l'homme du métier.

De préférence, la composition selon l'invention est formulée sous la forme de microsphères, également appelées sphéroïdes, préférentiellement regroupées en gélules selon une quantité correspondant par exemple à une prise unitaire.

On bénéficie ainsi des nombreux avantages de cette forme galénique, à savoir sur le plan médical une libération rapide et une grande biodisponibilité des principes actifs, et sur le plan pratique une grande facilité d'utilisation pour le patient et une forme peu fragile et très stable à long terme même dans des conditions de stockage difficiles.

Ces microsphères peuvent être réalisées par différents procédés.

Un premier procédé consiste par exemple à réaliser une couche périphérique contenant l'extrait végétal d'Hymenocardia acida autour d'un noyau neutre, formé par exemple d'un mélange de sucre et d'amidon ou d'un cristal de sucre, de mannitol ou de sorbitol.

La couche contenant l'extrait végétal d'Hymenocardia acida peut avantageusement être réalisée par enrobage, imprégnation, pulvérisation ou projection, le principe actif étant pour cela préférentiellement mélangé à un agent de montage ou à un liant.

Selon un deuxième procédé préférentiel, les microsphères ou sphéroïdes peuvent être formés par extrusion sphéronisation.

Pour cela, on commence par mélanger l'extrait végétal d'Hymenocardia acida à une substance absorbante et adsorbante, préférentiellement de type polymère naturel ou synthétique, présentant des propriétés plastiques compatibles avec les étapes d'extrusion et de sphéronisation de la suite du procédé.

Il s'agit par exemple de cellulose microcristalline, de cellulose microfine, d'un polymère cellulosique hydroxypropylé faiblement substitué, d'amidon, d'amidon modifié, de polysaccharides ou de toute autre substance ou mélange adapté.

Si le mélange est trop sec, un liquide d'humidification, aqueux ou non aqueux, peut également être ajouté pour obtenir une pâte homogène et malléable pouvant subir les étapes suivantes du procédé.

Le liquide d'humidification sert également de véhicule pour transporter et déposer le principe actif jusqu'au coeur de la substance absorbante et adsorbante, dans les microcavités du polymère.

Le procédé de fabrication consiste ensuite à extruder la masse humide à travers une filière à orifices calibrés. On obtient ainsi des filaments compacts, de section généralement cylindrique et définie, appelés « extrudats ».

Ces « extrudats » sont ensuite placés dans un appareil cylindrique appelé « sphéroniseur » contenant en sa partie inférieure un disque cannelé tournant à une vitesse variable et contrôlée. Sous l'effet de la force centrifuge exercée par la rotation du disque tournant, les « extrudats » se fragmentent régulièrement, puis se transforment en sphères par un effet de roulage-liage. On obtient ainsi les sphéroïdes souhaités.

Ces deux procédés peuvent comprendre en outre au moins une étape de séchage et/ou une étape de calibrage.

Le séchage peut se faire par chauffage léger par exemple à une température comprise entre 30 et 40°C ou par une simple exposition à l'air libre pendant une durée suffisante.

Enfin, les sphéroïdes résultants peuvent, de manière optionnelle, être revêtus en périphérie d'une pellicule extérieure en vue de les protéger par exemple de l'humidité extérieure, de la chaleur, des conditions agressives de l'organisme ou à effet retard.

Les présents inventeurs ont découvert que l'extrait végétal de feuilles d'Hymenocardia acida présente avantageusement, en plus de ses propriétés anti-hypertensives, des propriétés antibactériennes, notamment contre le Bacillus cereus, le Staphylococcus aureus et le Streptococcus mutans. Des propriétés anti-oxydantes et une activité anti-inflammatoire ont également été observées.

En ce qui concerne les propriétés anti-hypertensives de la composition selon l'invention, deux études thérapeutiques préliminaires ont conclu à une baisse modérée des pressions artérielles systoliques et/ou diastoliques chez des patients adultes hypertendus traités par un décocté de feuilles d'Hymenocardia acida.

Un essai clinique contrôlé multicentrique, en double aveugle, a démontré l'efficacité et la bonne tolérance d'une composition à base d'Hymenocardia acida dans le traitement de l'hypertension artérielle essentielle légère à modérée chez l'adulte.

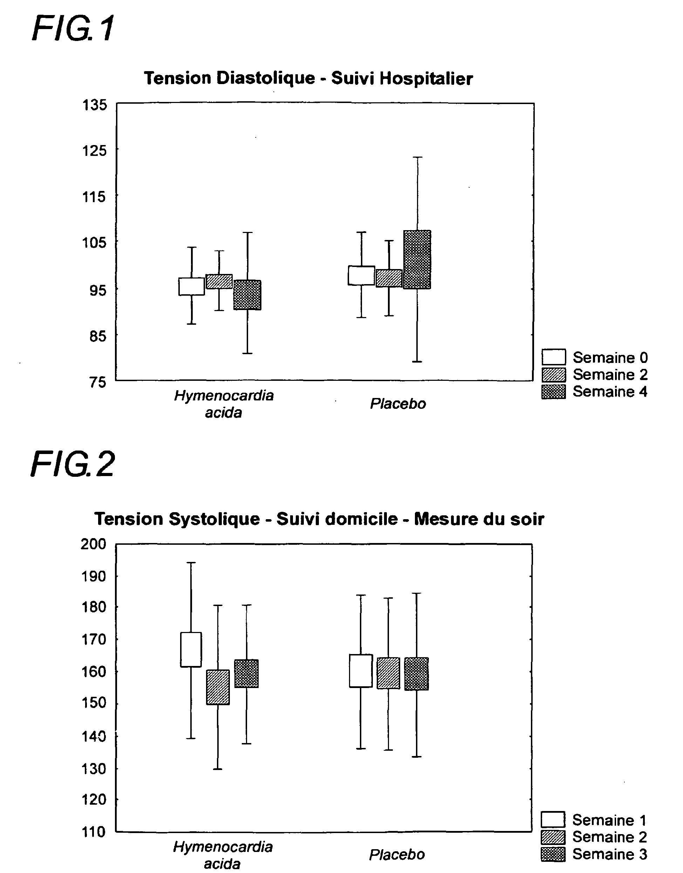

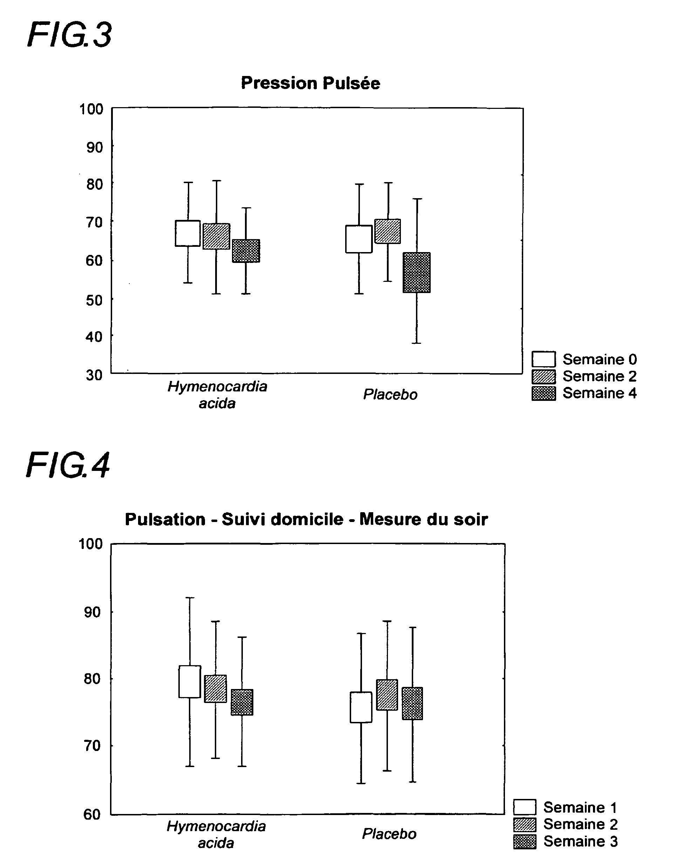

Les résultats de cet essai sont illustrés par les graphiques représentés sur les figures 1 à 4 accompagnant la présente demande.

Pour réaliser cet essai, on a sélectionné 36 patients âgés de moins de 40 ans, présentant des valeurs de tension systolique sur tension diastolique supérieures ou égales à 140/90 mmHg.

Ces patients ont été traités pendant quatre semaines, pour 17 d'entre eux avec une composition selon l'invention à base d'extrait de feuilles d'Hymenocardia acida et pour 19 d'entre eux avec une composition placebo.

Le traitement par voie orale a consisté pour chaque patient à prendre 6 gélules par jours, réparties en deux gélules le matin, le midi et le soir pendant quatre semaines.

Pour les patients traités avec la composition selon l'invention, ces gélules contenaient des microsphères réalisées selon le procédé préférentiel d'extrusion sphéronisation décrit ci-dessus à partir d'un extrait hydro-alcoolique de poudre de feuilles d'Hymenocardia acida obtenu par percolation exhaustive, avec un dosage correspondant à 350 ml d'extrait liquide d'Hymenocardia acida par gélule.

La tension systolique, la tension diastolique et la pulsation cardiaque de chaque patient ont été mesurées régulièrement tout au long de l'essai, à la fois à l'hôpital une fois par semaine au moyen d'un sphygmomanomètre standard et à domicile deux fois par semaine au moyen d'un tensiomètre électronique.

Les graphiques des figures 1 à 4 présentent les résultats obtenus.

Les valeurs mesurées ont fait l'objet de tests statistiques afin de pouvoir en déduire des résultats statistiquement significatifs. Ainsi, les tests de Kruskal-Wallis et de comparaisons multiples ont été appliqués pour comparer les groupes indépendants de données numériques. Le test Wilcoxon des paires associées a été utilisé pour comparer les groupes de données associées. Le test chi2 a été appliqué pour tester l'association entre les variables de deux catégories.

Comme on peut le voir sur les graphiques des figures 1 à 4, on a obtenu chez les patients traités avec la composition selon l'invention une baisse statistiquement significative de la tension systolique (p=0,017 à la semaine 2 et p=0.037 à la semaine 4) et de la pression pulsée (p=0,02) qui correspond à la différence entre la pression artérielle systolique et la pression artérielle diastolique.

De même une baisse significative de la pulsation cardiaque a été enregistrée chez les patients traités avec la composition selon l'invention.

Avantageusement, lors de cet essai clinique, on n'a observé que peu d'effets secondaires provoqués par la composition selon l'invention.

Une polyurie a été ressentie par 1/17 patient traité par la composition selon l'invention et par 1/19 patient traité par le placebo, une somnolence a été ressentie par 2/19 patients traités par le placebo, une diarrhée a été ressentie par 1/19 patient traité par le placebo et une toux a été ressentie par 1/17 patient traité par la composition selon l'invention.

Les effets secondaires rapportés pour la composition selon l'invention sont donc bénins et peu fréquents. Ils sont même moins nombreux que ceux rapportés par les patients traités par le placebo.

En plus de l'extrait végétal d'Hymenocardia acida, la composition selon l'invention peut contenir un ou plusieurs autres composés ou excipients appropriés tels que par exemple des extraits d'une ou plusieurs autres plantes, des vitamines, des minéraux, des oligoéléments, des composés augmentant l'efficacité du principe actif, le protégeant ou aidant à sa libération rapide, des arômes, des agents de montage, des composés liants, des composés glissants, des composés lubrifiants, des tensioactifs, des sucres, du lactose, du sorbitol, du mannitol, des amidons ou amidons modifiés, des maltodextrines, des carbonates, des citrates, de la gélatine, de la polyvinylpyrrolidone, des dérivés cellulosiques, de la carboxyméthylcellulose sodique réticulée ou tout autre composé approprié.

Par exemple, la composition anti-hypertensive de l'invention peut contenir en outre un extrait végétal d'une ou plusieurs plantes riches en silice présentant un rôle bénéfique sur la souplesse des parois du tissu artério-veineux. Il peut s'agir notamment d'un extrait de prèle et/ou de bambou.

Selon un mode de réalisation préférentiel de l'invention, la composition anti-hypertensive de l'invention contient du sélénium.

Le sélénium est un oligo-élément indispensable au fonctionnement du cεur, qui présente des propriétés anti-oxydantes et un rôle protecteur du système cardio-vasculaire.

Ses effets bénéfiques s'ajoutent ainsi aux propriétés anti-oxydantes des feuilles d'Hymenocardia acida en vue d'éviter d'éventuelles défaillances cardiaques du patient.

Une synergie entre l'extrait d'Hymenocardia acida et le sélénium est espérée, ce qui pourrait conduire à une augmentation de l'efficacité de la composition selon l'invention et/ou à une diminution des doses employées pour les principes actifs.

Dans la composition selon l'invention, le sélénium est apporté par exemple sous la forme de sélénite de sodium, en quantité préférentiellement comprise entre 17.5 et 21,9 □g par gélule de 350 mg, ce qui correspond à une quantité comprise entre environ 8 et 10 □g d'équivalent sélénium par gélule de 350 mg.

L'ajout d'une quantité dosée de sélénium permet en outre, en plus de son action bénéfique pour la santé, de contrôler la qualité de la fabrication en jouant le rôle d'un traceur dont la quantité peut être mesurée avec précision.

Il permet également de contrôler la stabilité au fil du temps des formes galéniques réalisées à partir de la composition.

Les exemples suivants permettront de mieux illustrer l'invention.

Des sphéroïdes ont été réalisés par le procédé d'extrusion sphéronisation décrit précédemment, à partir d'un extrait hydro-alcoolique de feuilles d'Hymenocardia acida, préparé selon la pharmacopée européenne, avec une teneur en éthanol de 70% V/V et un résidu sec d'environ 10.62 %. Ces sphéroïdes ont été rassemblés en gélules de 350 mg.

### Exemple 1 :

| Pour une gélule de 350 mg : | |
|---|---|
| • extrait d'Hymenocardia acida | 350 ml |
| • microcellulose | q.s. |

### Exemple 2 :

| Pour une gélule de 350 mg : | |
|---|---|
| • extrait d'Hymenocardia acida | 350 ml |
| • sélénite de sodium, en équivalent sélénium | 8 □g |
| • microcellulose | q.s. |

### Exemple 3 :

| Pour une gélule de 350 mg : | |
|---|---|
| • extrait d'Hymenocardia acida | 315 ml |
| • extrait liquide de prèle | 35 ml |
| • microcellulose | q.s. |

### Exemple 4 :

| Pour une gélule de 350 mg : | |
|---|---|
| • extrait d'Hymenocardia acida | 315 ml |
| • extrait liquide de bambou | 35 ml |
| • microcellulose | q.s. |

Les sphéroïdes obtenus ont été regroupés en gélules de 350 mg correspondant à une dose unitaire de composition, pour une posologie préférentielle de trois à six gélules par jour.

De manière évidente, l'invention ne se limite pas aux modes de réalisation préférentiels décrits précédemment, l'homme du métier pouvant y apporter de nombreuses modifications et imaginer d'autres variantes sans sortir ni de la portée, ni du cadre de l'invention.

## Revendications

1. Utilisation d'un extrait végétal d'Hymenocardia acida pour la fabrication d'une composition pharmaceutique pour la prévention et le traitement de l'hypertension.

2. Utilisation d'un extrait végétal d'Hymenocardia acida selon la revendication précédente **caractérisée en ce que** l'extrait végétal d'Hymenocardia acida est obtenu à partir de feuilles d'Hymenocardia acida.

3. Utilisation d'un extrait végétal d'Hymenocardia acida selon la revendication précédente **caractérisée en ce que** l'extrait végétal d'Hymenocardia acida est un extrait hydro-alcoolique de feuilles d'Hymenocardia acida.

4. Utilisation d'un extrait végétal d'Hymenocardia acida selon la revendication précédente **caractérisée en ce que** l'extrait végétal d'Hymenocardia acida est une teinture mère d'Hymenocardia acida avec une teneur en éthanol d'environ 70% V/V.

5. Utilisation d'un extrait végétal d'Hymenocardia acida selon l'une quelconque des revendications précédentes **caractérisée en ce que** la composition pharmaceutique contient en outre un ou plusieurs composés ou excipients choisis parmi les extraits de plantes, les vitamines, les minéraux, les oligoéléments, les composés augmentant l'efficacité du principe actif, le protégeant ou aidant à sa libération rapide, les arômes, les agents de montages, les composés liants, les composés glissants, les composés lubrifiants, les tensioactifs, les sucres, le lactose, le sorbitol, le mannitol, les amidons et amidons modifiés, les maltodextrines, les carbonates, les citrates, la gélatine, la polyvinylpyrrolidone, les polysaccharides, les dérivés cellulosiques, dont la carboxyméthylcellulose sodique réticulée, la cellulose microcristalline, la cellulose microfine et les polymères cellulosiques hydroxypropylés faiblement substitués.

6. Utilisation d'un extrait végétal d'Hymenocardia acida selon la revendication précédente **caractérisée en ce que** la composition pharmaceutique contient du sélénium.

7. Utilisation d'un extrait végétal d'Hymenocardia acida selon la revendication précédente **caractérisée en ce que** le sélénium est apporté sous la forme de sélénite de sodium.

8. Utilisation d'un extrait végétal d'Hymenocardia acida selon l'une quelconque des revendications 5 à 7 **caractérisée en ce que** la composition pharmaceutique contient un extrait végétal d'une plante riche en silice.

9. Utilisation d'un extrait végétal d'Hymenocardia acida selon la revendication précédente **caractérisée en ce que** la composition pharmaceutique contient un extrait de prèle ou de bambou.

10. Utilisation d'un extrait végétal d'Hymenocardia acida selon l'une quelconque des revendications précédentes **caractérisée en ce que** la composition pharmaceutique se présente sous une forme galénique d'infusettes, de microsphères, de comprimés, de comprimés orodispersibles, de capsules molles, de granules, de gélules, de poudre, d'ampoules ou de sirop.

11. Utilisation d'un extrait végétal d'Hymenocardia acida selon la revendication précédente **caractérisée en ce que** la composition pharmaceutique se présente sous une forme galénique d'infusettes contenant dix à vingt grammes de feuilles d'Hymenocardia acida grossièrement broyées.

12. Utilisation d'un extrait végétal d'Hymenocardia acida selon la revendication 10 **caractérisée en ce que** la composition pharmaceutique se présente sous une forme galénique de microsphères réalisées par un procédé d'extrusion sphéronisation.

13. Utilisation d'un extrait végétal d'Hymenocardia acida selon la revendication précédente **caractérisée en ce que** les microsphères sont regroupées en gélules.

14. Utilisation d'un extrait végétal d'Hymenocardia acida selon l'une quelconque des revendications précédentes dans la formulation d'une composition pharmaceutique pour le traitement des cas d'hypertension faible et modérée correspondant aux stades 1 et 2 de la classification, ou les cas de pré-hypertension.

## Claims

1. Use of a vegetal extract of Hymenocardia acida for producing a pharmaceutical composition for the prevention and treatment of the hypertension.

2. Use of a vegetal extract of Hymenocardia acida according to the previous claim **characterized in that** the vegetal extract of Hymenocardia acida is obtained from leaves of Hymenocardia acida.

3. Use of a vegetal extract of Hymenocardia acida according to the previous claim **characterized in that** the vegetal extract of Hymenocardia acida is a hydroalcoholic extract of leaves of Hymenocardia acida.

4. Use of a vegetal extract of Hymenocardia acida according to the previous claim **characterized in that** the vegetal extract of Hymenocardia acida is a mother tincture of Hymenocardia acida with an ethanol content of about 70% v/v.

5. Use of a vegetal extract of Hymenocardia acida according to any previous claim **characterized in that** the pharmaceutical composition furthermore contains one or more compounds or vehicles selected among plant extracts, vitamins, minerals, oligoelements, compounds augmenting the efficiency of the active principle by protecting it or helping its fast release, flavors, composition agents, binding compounds, slippery compounds, lubricant compounds, tensioactives, sugars, lactose, sorbitol, mannitol, starches and modified starches, maltodextrines, carbonates, citrates, gelatin, polyvinylpyrrolidone, polysaccarides, cellulosics including reticulate sodium carboxymethylcellulose, microcrystalline cellulose, microfine cellulose and low substituted cellulosic hydroxypropyle polymeres.

6. Use of a vegetal extract of Hymenocardia acida according to the previous claim **characterized in that** the pharmaceutical composition contains selenium.

7. Use of a vegetal extract of Hymenocardia acida according to the previous claim **characterized in that** the selenium is supplied in form of sodium selenite.

8. Use of a vegetal extract of Hymenocardia acida according to any of the claims 5 to 7 **characterized in that** the pharmaceutical composition contains a vegetal extract of a plant rich in silica.

9. Use of a vegetal extract of Hymenocardia acida according to the previous claim **characterized in that** the pharmaceutical composition contains an extract of horsetail or bamboo.

10. Use of a vegetal extract of Hymenocardia acida according to any previous claim **characterized in that** the pharmaceutical composition is in a galenic form of tea bags, of microspheres, of pills, of orodispersible pills, of soft capsules, of granules, of capsules, of powder, of ampoules or syrup.

11. Use of a vegetal extract of Hymenocardia acida according to the previous claim **characterized in that** the pharmaceutical composition is in a galenic form of tea bags containing ten to twenty grammes of coarsely crushed Hymenocardia acida leaves.

12. Use of a vegetal extract of Hymenocardia acida according to claim 10 **characterized in that** the pharmaceutical composition is in a galenic form of microspheres realized by an extrusion-spheronisation process.

13. Use of a vegetal extract of Hymenocardia acida according to the previous claim **characterized in that** the microspheres are grouped together in capsules.

14. Use of a vegetal extract of Hymenocardia acida according to any of the preceding claims in the formulation of a pharmaceutical composition for the treatment of weak and moderate hypertension cases corresponding to the stages 1 and 2 of the classification, or of prehypertension cases.

## Patentansprüche

1. Verwendung eines Pflanzenextrakts aus Hymenocardia acida zur Herstellung einer pharmazeutischen Zusammensetzung zur Vorbeugung und Behandlung des Bluthochdrucks.

2. Verwendung eines Pflanzenextrakts aus Hymenocardia acida nach dem vorherigen Anspruch, **gekennzeichnet dadurch, dass** der Pflanzenextrakt aus Hymenocardia acida aus Blättern von Hymenocardia acida erhalten wird.

3. Verwendung eines Pflanzenextrakts aus Hymenocardia acida nach dem vorherigen Anspruch, **gekennzeichnet dadurch, dass** der Pflanzenextrakt aus Hymenocardia acida ein hydroalkoholischer Extrakt aus Blättern von Hymenocardia acida ist.

4. Verwendung eines Pflanzenextrakts aus Hymenocardia acida nach dem vorherigen Anspruch, **gekennzeichnet dadurch, dass** der Pflanzenextrakt aus Hymenocardia acida eine Muttertinktur aus Hymenocardia acida mit einem Äthanolgehalt von etwa 70% v/v ist.

5. Verwendung eines Pflanzenextrakts aus Hymenocardia acida nach einem der vorherigen Ansprüche, **gekennzeichnet dadurch, dass** die pharmazeutische Zusammensetzung außerdem eine oder mehrere Zusammensetzungen oder Trägerstoffe enthält, ausgewählt aus den folgenden: Pflanzenextrakte, Vitamine, Mineralien, Oligoelemente, Zusammensetzungen, die die Wirksamkeit des aktiven Prinzips erhöhen, indem sie es schützen oder zu seiner schnellen Freisetzung beitragen, Aromen, Zusammensetzungs-Wirkstoffe, Bindeelemente, glatte Mischungen, Gleitmittel, Tenside, Zucker, Milchzucker, Sorbitol, Mannitol, Stärken und modifizierte Stärken, Maltodextrine, Karbonate, Zitrate, Gallert, Polyvinylpyrrolidon, Polysaccharide, Zelluloseharze, darunter vernetzte Natrium-Carboxymethylcellulose, mikrokristalline Cellulose, mikrofeine Cellulose und niedrig substituierte Hydroxypropyl-Cellulosepolymere.

6. Verwendung eines Pflanzenextrakts aus Hymenocardia acida nach dem vorherigen Anspruch, **gekennzeichnet dadurch, dass** die pharmazeutische Zusammensetzung Selen enthält.

7. Verwendung eines Pflanzenextrakts aus Hymenocardia acida nach dem vorherigen Anspruch, **gekennzeichnet dadurch, dass** das Selen in Form von Natriumselenit beigeführt wird.

8. Verwendung eines Pflanzenextrakts aus Hymenocardia acida nach einem beliebigen der Patentansprüche 5 bis 7, **gekennzeichnet dadurch, dass** die pharmazeutische Zusammensetzung einen Pflanzenextrakt aus einer kieselsäurereichen Pflanze enthält.

9. Verwendung eines Pflanzenextrakts aus Hymenocardia acida nach dem vorherigen Anspruch, **gekennzeichnet dadurch, dass** die pharmazeutische Zusammensetzung einen Extrakt aus Schachtelhalm oder Bambus enthält.

10. Verwendung eines Pflanzenextrakts aus Hymenocardia acida nach einem der vorherigen Ansprüche, **gekennzeichnet dadurch, dass** die pharmazeutische Zusammensetzung eine galenischen Form von Aufgussbeuteln, Mikrokugeln, Tabletten, Schmelztabletten, Weichkapseln, Körnchen, Kapseln, Puder, Ampullen oder Sirup hat.

11. Verwendung eines Pflanzenextrakts aus Hymenocardia acida nach dem vorherigen Anspruch, **gekennzeichnet dadurch, dass** die pharmazeutische Zusammensetzung eine galenische Form von Aufgussbeuteln hat, die zehn bis zwanzig Gramm grob zerstampfte Hymenocardia-acida-Blätter enthalten.

12. Verwendung eines Pflanzenextrakts aus Hymenocardia acida nach Anspruch 10, **gekennzeichnet dadurch dass** die pharmazeutische Zusammensetzung einer galenische Form von Mikrokugeln hat, die mit einem Fliesspress-Rondier-Verfahren hergestellt werden.

13. Verwendung eines Pflanzenextrakts aus Hymenocardia acida nach dem vorherigen Anspruch, **gekennzeichnet dadurch, dass** die Mikrokugeln in Kapseln gruppiert sind.

14. Verwendung eines Pflanzenextrakts aus Hymenocardia acida nach einem beliebigen der vorherigen Ansprüche in der Formel einer pharmazeutischer Zusammensetzung für die Behandlung von Fällen geringen und mäßigen Bluthochdrucks entsprechend den Stadien 1 und 2 der Klassifizierung, oder von Fällen von Prä-Hypertonie.
